# EUROPÄISCHE PATENTSCHRIFT

(11) **EP 0 804 540 B1**
(45) Veröffentlichungstag und Bekanntmachung des Hinweises auf die Patenterteilung: **05.04.2000**
(21) Anmeldenummer: 96900906.7
(22) Anmeldetag: 05.01.1996
(51) Int. Cl.: C12M 1/22, B01L 9/00, F16M 13/00

(54) **HALTEVORRICHTUNG FÜR PETRISCHALEN**
PETRI DISH HOLDER
SUPPORT POUR BOITES DE PETRI

(30) Priorität: 27.01.1995 DE 19502520
(43) Veröffentlichungstag der Anmeldung: 05.11.1997
(73) Patentinhaber: Orgamed Laborsysteme Vertriebsgesellschaft mb H, 71394 Kernen (DE)
(72) Erfinder: ZAUSER, Kurt, D-71394 Kernen (DE); ZAUSER, Thomas, D-81394 Kernen (DE)
(74) Vertreter: Dreiss, Fuhlendorf, Steimle & Becker
(86) Internationale Anmeldenummer: EP9600029
(87) Internationale Veröffentlichungsnummer: WO9623055

(56) Entgegenhaltungen:
- DE-C- 4 300 231
- US-A- 4 143 765
- US-A- 4 321 330
- PATENT ABSTRACTS OF JAPAN vol. 012, no. 155 (C-494), 12.Mai 1988 & JP,A,62 269757 (HITACHI ELECTRONICS ENG CO LTD), 24.November 1987,

## Beschreibung

Die Erfindung betrifft eine Haltevorrichtung für Petrischalen *gemäß dem Oberbegriff des Anspruchs 1.*

Petrischalen sind in der Regel flache Kunststoffschalen mit einem Deckel und werden bspw. zur Züchtung und zum Bebrüten von Bakterien- und Pilzkulturen verwendet. Diese Kulturen werden nicht nur in einer einzigen, sondern in einer Vielzahl derartiger Petrischalen angezüchtet, wobei an den Kulturen in einer vorgegebenen Reihenfolge eine Vielzahl Untersuchungen durchgeführt werden. Wichtig dabei ist, daß eine vorgegebene Reihenfolge während des gesamten Untersuchungsvorganges, der mehrere Tage andauern kann, eingehalten wird. Dabei müssen die Petrischalen permanent zwischen dem Brutschrank und den Arbeitsplätzen hin- und hertransportiert werden, wobei darauf geachtet werden muß, daß die Reihenfolge der in der Regel aufeinander gestapelten Petrischalen eingehalten wird. Bei diesem Transport können lediglich immer nur wenige der lose gestapelten Petrischalen gefahrlos transportiert werden. Da sich während des gesamten Untersuchungsvorganges die Kennung der Schalen nicht sichtbar ist, bedarf es eines hohen organisatorischen Aufwandes, um die einzelnen Petrischalen, die in der Regel unterschiedliche Zyklen durchlaufen haben, in der vorgegebenen arbeitsplatzbedingten Reihenfolge zu halten. Insbesondere dann, wenn aufgrund weiterer Untersuchungen besonders zu handhabende Teilmengen entstehen, ist die Gefahr groß, daß einzelne Petrischalen miteinander vertauscht werden. Auf diese Weise können sich Fehler in die Untersuchungsergebnisse einschleichen. Eine weitere wichtige Gefahr besteht darin, daß trotz vorsichtigen Hantierens die gestapelten Petrischalen umfallen und die Arbeitstische bzw. den Boden mit pathogenen Bakterien kontaminieren.

Aus der DE 43 00 231 C1 sind Petrischalen bekannt geworden, die mit einer besonderen Verbindungseinheit ausgestattet sind, so daß sie einerseits aufeinander steckbar sind, andererseits in eine Haltevorrichtung einklemmbar sind und zudem aus ihrem Verbund herausgeschwenkt werden können. Als nachteilig hat sich bei diesen Petrischalen herausgestellt, daß deren Aufbau äußerst kompliziert ist und die Petrischalen, auch in der Massenfertigung, daher relativ teuer sind.

Mit der DE 26 28 344 A1 ist ein Tablett zur Aufnahme mehrerer Petrischalen bekannt geworden. Zwar sind hier die Petrischalen verliersicher gehalten, jedoch kann dieses Tablett eine nur sehr geringe Anzahl von Petrischalen aufnehmen, die außerdem umständlich aus dem Tablett zu entfernen sind.

Mit der EP 447 893 A2 ist ein Haltesystem für einen Stapel von Petrischalen bekannt geworden, mit dem der Stapel zwar problemlos gehalten werden kann, eine Entnahme einzelner Petrischalen aus dem Stapel jedoch nicht oder nur sehr schwierig möglich ist.

Aus der US 4,143,765 ist Haltevorrichtung für Petrischalen bekannt geworden, die einen Grundkörper zur Aufnahme mehrerer Petrischalen aufweist. Die Petrischalen werden dabei von dem Grundkörper weitgehend umgriffen, der im Querschnitt um die Petrischalen weitgehend C-förmig ausgebildet ist und die einzelnen Petrischalen über einen Winkel von mehr als 180° ihres Umfanges umgreift. Eine solche Haltevorrichtung weißt den Nachteil auf, daß der Grundkörper relativ sperrig ist, unabhängig davon, wieviel Petrischalen in der Haltevorrichtung gehalten oder transportiert werden sollen.

Der Erfindung liegt daher die Aufgabe zugrunde, eine Haltevorrichtung für Petrischalen bereitzustellen, die einerseits eine große Anzahl derartiger Schalen aufnimmt, andererseits einzelne Schalen oder der gesamte Stapel aus der Haltevorrichtung problemlos entnehmbar ist und außerdem die Haltevorrichtung relativ preiswert ist und einen Schutz für die Schalen gegen Herausfallen bietet. Eine weitere Aufgabe der Erfindung ist, daß die Haltevorrichtung möglichst klein und platzsparend ausgeführt sein soll.

Diese Aufgabe wird mit einer Haltevorrichtung gelöst, die die Merkmale des Anspruchs 1 aufweist.

Erfindungsgemäß weist der Grundkörper auf der zur Petrischalen aufnehmenden Seite abgewandten Seite eine Verbindungseinrichtung zur Ankopplung einer oder mehrerer weiterer Haltevorrichtungen auf. Über diese Verbindungseinrichtung können je nach Anzahl der zu lagernden und zu transportierenden Petrischalen entsprechend viele Grundkörper zu einer Halteeinheit zusammengefügt werden, wobei die Halteeinheit ein entsprechend Vielfaches der Petrischalen aufnimmt. Auf diese Weise können mit der Halteeinheit z.B. bis zu 84 Petrischalen gefahrlos transportiert und gelagert werden.

Die im wesentlichen C-förmige Ausgestaltung hat den besonderen Vorteil, daß eine oder mehrere Petrischalen (mit Deckel) problemlos radial in diese Haltevorrichtung eingeschoben werden können, ohne daß an der Haltevorrichtung besondere Vorkehrungen zur Halterung oder Sicherung der Schalen getroffen werden müssen. Vor allem muß diese Haltevorrichtung weder geöffnet noch geschlossen werden, noch muß die Haltevorrichtung zerlegt oder zur Aufnahme der Schalen wieder zusammengebaut werden. Bei der erfindungsgemäßen Vorrichtung kann eine einzige Schale, in der Regel jedoch ein Stapel von 21 Petrischalen auf einmal radial in den Grundkörper eingeschoben werden und wird von diesem umgriffen und gehalten. Dabei wird der Umfang der Petrischalen über einen Winkel von 182° bis 220°, bevorzugt über einen Winkel von 190° umgriffen und dabei geringfügig hintergriffen. Diese Hintergriff verhindert einerseits ein Herausfallen der Petrischalen aus der Haltevorrichtung, und erlaubt andererseits einen problemlosen Transport der Haltevorrichtung mit eingesetzten Schalen.

Auf diese Weise wird eine hohe Sicherheit gegen Vertauschen einzelner Petrischalen geschaffen, so daß die Reihenfolge bei der Untersuchung der einzelnen Bakterienkulturen mühelos und ohne besondere organisatorische Maßnahmen eingehalten werden kann. Aus der Haltevorrichtung kann in einem Arbeitsgang der gesamte Stapel entnommen werden und dieser Stapel kann nach seiner Abarbeitung in der gleichen Reihenfolge der Schalen wieder in die Haltevorrichtung eingesetzt werden, so daß die Petrischalen wieder gegen Verrutschen, Vertauschen u.dgl. gesichert sind. Der Schalenstapel kann auch folgendermaßen abgearbeitet werden: Die oberste Schale wird entnommen und nach Beendigung der Arbeit umgekehrt, d.h. mit der Unterseite nach oben bzw. mit der Oberseite nach unten in eine neue Haltevorrichtung eingesetzt. Die nächste Schale wird nach Beendigung der Arbeit ebenfalls umgekehrt in die neue Haltevorrichtung auf die zuvor eingelegte Schale aufgesetzt. Nach Abarbeitung des Stapels wird die vollständig angefüllte Haltevorrichtung umgedreht und der neue Stapel befindet sich wieder in der korrekten Lage. Die Haltevorrichtung bietet außerdem durch das weite Umgreifen der Petrischalen einen guten Schutz gegen Bruch und zur Vermeidung von Kontamination.

Bei einer besonders bevorzugten Ausführungsform ist die Verbindungseinrichtung eine Zapfen-Loch-Verbindung. Es ist jedoch auch eine Nut-Feder-Verbindung, z.B. eine Schwalbenschwanz-Verbindung denkbar. Diese Zapfen-Loch-Verbindung erlaubt ein Zerlegen der Halteeinheit in die einzelnen Haltevorrichtungen (z.B. zu Reinigungszwecken) und ein anschließender Zusammenbau ohne Werkzeug. Dabei können die beiden Verbindungselemente der Verbindungseinrichtung in einem Winkel von 0°, 60°, 90°, 108°, usw. zueinander stehen. Somit können 2, 3, 4, 5, oder mehr Haltevorrichtungen zu einer einzigen Halteeinheit verbunden werden. Das Verbinden und Lösen der Haltevorrichtungen voneinander kann ohne oder mit eingesetzten Petrischalen erfolgen.

Bei einer bevorzugten Ausführungsform bilden die miteinander verbundenen Haltevorrichtungen eine Aufnahme für ein Gestell o.dgl. Diese Aufnahme sieht z.B. eine zylinderförmige Ausnehmung vor, in die ein vertikaler Stab eines Drehtellers eingeschoben werden kann. Auf diese Weise kann die Halteeinheit mit den darin vorgesehenen Petrischalen bequem am Arbeitsplatz bedient werden, wobei jeweils die zu bearbeitende Seite durch Drehen der gesamten Einheit herangeholt werden kann.

Eine Weiterbildung sieht vor, daß zwei oder mehr derartiger Haltevorrichtungen übereinander stapelbar sind. Auf diese Weise kann die Aufnahmekapazität der gesamten Halteeinheit vervielfacht werden. Der Platzbedarf an den Arbeitsplätzen wird auf diese Weise erheblich reduziert, wobei die Petrischalen nach wie vor sicher gehalten werden.

Um ein axiales Herausrutschen der Petrischalen aus der Haltevorrichtung zu verhindern, ist sie mit einem Boden und/oder einem Deckel versehen.

Das Material, aus dem die Haltevorrichtung hergestellt ist, weist die Eigenschaft auf, daß es zumindest geringfügig elastisch ist. Bei einem Kunststoff oder einem Metall aus Federstahl o.dgl. ist dies der Fall. Außerdem ist das Material der Haltevorrichtung temperaturbeständig, so daß die Petrischalen zusammen mit der Haltevorrichtung in einem Brutschrank abgestellt werden können. Um die Haltevorrichtung problemlos reinigen und desinfizieren zu können, ist das Material zumindest bis 150° C temperaturbeständig, und beständig gegen Säuren, Laugen, Alkohole und andere Desinfektionsmittel. Auf jeden Fall sollte das Material z.B. in einem Autoklaven einer Temperatur von 125° C über einen Zeitraum von ca. 25 Minuten ohne weiteres Stand halten.

Außerdem ist das Material einfärbbar oder weist bestimmte Farben oder Kodierungen auf. Durch die farbliche Kennzeichnung der einzelnen Haltevorrichtungen können einzelne Arbeitsstadien bzw. Untersuchungsfortschritte an den Kulturen gekennzeichnet werden. Auf diese Weise ist sofort erkennbar, welche Arbeitsschritte an den Kulturen bereits durchgeführt worden sind und welche als nächste durchzuführen sind. Die Petrischalen werden hierfür aus der einen Haltevorrichtung mit der einen farblichen Kennzeichnung entnommen und nach Durchführung des Arbeitsschrittes in eine Haltevorrichtung eingesetzt, die eine entsprechend andere Färbung oder Kennzeichnung aufweist.

Bei einer anderen Ausführungsform ist die Haltevorrichtung beschriftbar, so daß der jeweilige Stand der Untersuchung auf der Haltevorrichtung gekennzeichnet werden kann. Diese Beschriftung ist problemlos wieder entfernbar. Bei weiteren Ausführungsformen ist vorgesehen, daß die Haltevorrichtung ein Kennzeichnungfeld aufweist, an welchem Markierungen, Notizen o.dgl. befestigbar sind.

Um einen einfachen und problemlosen Transport zu ermöglichen, weist die Haltevorrichtung eine Tragevorrichtung auf oder es ist eine Tragevorrichtung an der Haltevorrichtung befestigbar. Eine einfache Ausgestaltung sieht dabei bügelförmige Tragegriffe vor.

Weitere Vorteile der Erfindung sind, daß die Haltevorrichtung bezüglich ihrer zur Längsachse orthogonalen Mittelebene einen symmetrischen Aufbau aufweist. Auf diese Weise kann die Haltevorrichtung problemlos umgedreht und weiterverwendet werden. Bevorzugt sind alle Grundkörper gleich ausgebildet, was den Vorteil hat, daß ein einziges Spritzgußwerkzeug erforderlich ist. Außerdem sind die einzelnen Grundkörper einteilig ausgebildet.

Weitere Vorteile, Merkmale und Einzelheiten der Erfindung ergeben sich aus der nachfolgenden Beschreibung, in der unter Bezugnahme auf die Zeichnung besonders bevorzugte Ausführungsbeispiele im einzelnen beschrieben ist. In der Zeichnung zeigen:
- Figur 1: einen Querschnitt durch eine Haltevorrichtung;
- Figur 2: mehrere miteinander verbundene Haltevorrichtungen;
- Figur 3: eine perspektivische Ansicht der Haltevorrichtung gemäß Figur 1; und
- Figur 4: einen Teilschnitt durch eine andere Ausführungsform der Haltevorrichtung mit eingesetztem Eckelement.

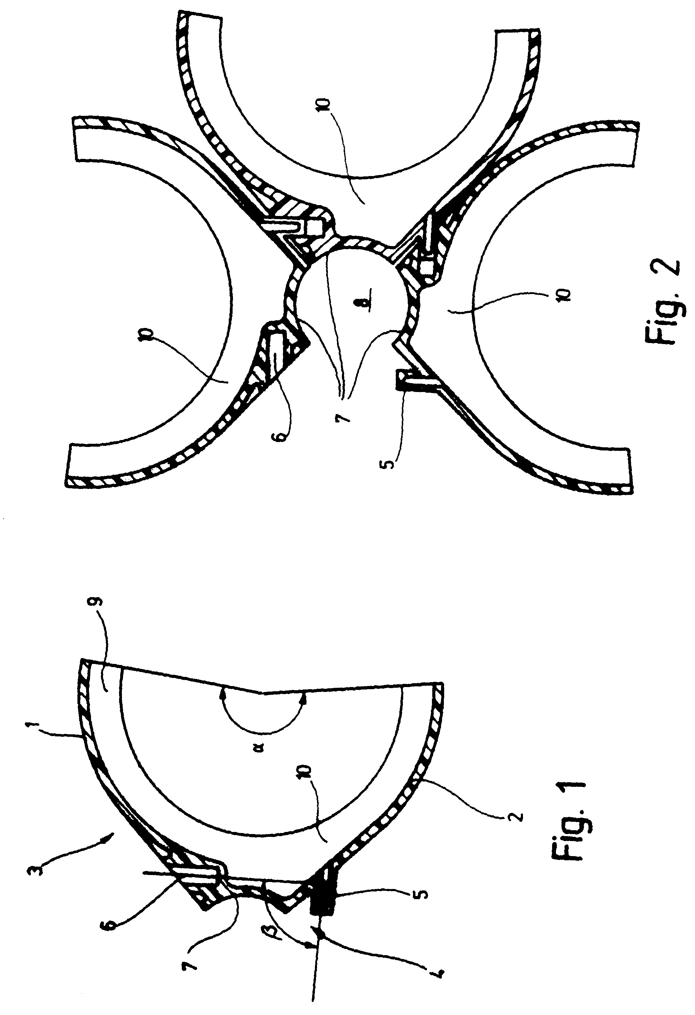

In der Figur 1 ist ein Querschnitt einer Ausführungsform der erfindungsgemäßen Haltevorrichtung dargestellt, die im wesentlichen C-förmig ausgebildet ist. Dabei bilden die beiden Schenkel 1 und 2 des Grundkörpers 3 einen Winkel α von 190°. Dies erlaubt ein sicheres Hintergreifen einer oder mehrerer nicht dargestellter Petrischalen, die einen Außendurchmesser aufweisen, der im wesentlichen dem Innendurchmesser der teilkreisförmigen Schenkel 1 und 2 entspricht. Die Schenkel 1 und 2 bilden an ihren einander zugewandten Enden eine Verbindungseinrichtung 4, über die vier Haltevorrichtungen zu einer Halteeinheit zusammengefügt werden können. Die Verbindungseinrichtung 4 weist einen Zapfen 5 und eine Bohrung 6 auf, wobei die Achsen des Zapfens 5 und der Bohrung 6 in einem Winkel β von 90° zueinander stehen. Auf diese Weise kann der Zapfen 5 mit einer Bohrung 6 einer benachbarten Haltevorrichtung und der Zapfen einer anderen benachbarten Haltevorrichtung mit der Bohrung 6 verbunden werden. Diese Anordnung ist in der Figur 2 dargestellt. Aus der Figur 2 ist außerdem ersichtlich, daß die Verbindungseinrichtung 4 eine Ausnehmung 7 aufweist, die zusammen mit weiteren Ausnehmungen 7 benachbarter Haltevorrichtungen (insgesamt 4 Stück) eine Aufnahme 8, z.B. für einen vertikalen Stab eines Drehtellers bildet.

Aus den Figuren 1 und 2 ist außerdem erkennbar, daß die Innenseite der Schenkel 1 und 2 mit einem radial nach innen vorstehenden Rand 9 versehen sind, der einen Boden 10 für in die Haltevorrichtung eingesetzte Petrischalen bildet, der ein Herausfallen der Petrischalen in axialer Richtung verhindert.

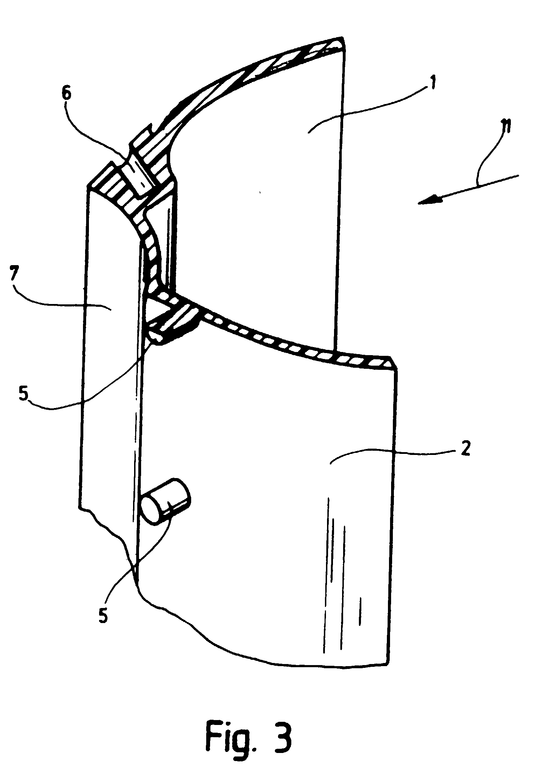

Die Figur 3 zeigt eine abgebrochene perspektivische Darstellung der Haltevorrichtung der Figur 1, wobei deutlich erkennbar ist, daß in diese Haltevorrichtung bequem ein ganzer Stapel Petrischalen radial, d.h. in Richtung des Pfeils 11 einsetzbar bzw. entgegen der Richtung des Pfeils 11 aus der Haltevorrichtung herausnehmbar ist. Der eingesetzte Stapel wird von den freien Enden der Schenkel 1 und 2 der Haltevorrichtung geringfügig hintergriffen und somit gegen ein radiales Herausfallen festgehalten. Um mehrere derartiger Haltevorrichtungen sicher miteinander zu einer Halteeinheit zu verbinden, sind mehrere, insgesamt 6 Stück, der Zapfen 5 und Bohrungen 6 übereinander angeordnet. Auf diese Weise werden die einzelnen Haltevorrichtungen über deren gesamte Länge sicher miteinander verbunden.

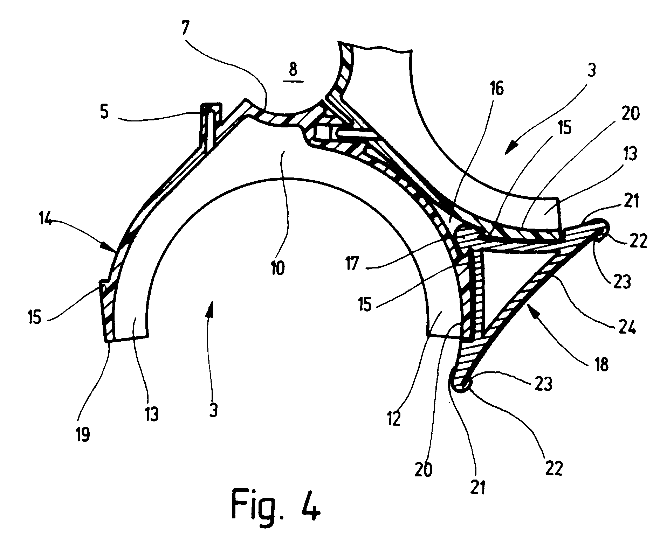

In der Figur 4 ist eine Variante der Haltevorrichtung dargestellt, bei die die freien Enden 12 und 13 der Grundkörper 3 an ihren Außenseiten 14 mit Haltearmen 15 versehen sind. Zwei einander gegenüberliegende Haltenasen 15 zweier Grundkörper 3 bilden eine Rastaufnahme 16 für einen Rastzapfen 17 eines Eckelements 18.

Dieses Eckelement 18 kann sich über einen Teil der Höhe oder über die gesamte Höhe der Haltevorrichtung erstrecken. Sie weist eine im wesentlichen dreieckförmige Gestalt auf und ist so geformt, daß sie sich an die Außenseiten 15 der Grundkörper 3 anschmiegt. Die Stirnfläche 14 der freien Enden 12, 13 der Grundkörper ist übergriffen, so daß die Innenfläche 20, an der die Petrischalen anliegen, durch einen Fortsatz 21 verlängert wird. Das Eckelement 18 ist hohl ausgebildet, wodurch die Elastizität erhöht wird. Durch die Fortsätze 21 wird die Möglichkeit geschaffen, auch im Durchmesser kleinere Petrischalen mit Sicherheit aufzunehmen, da der Umschlingungswinkel z.B. auf 210° erhöht wird. Außerdem weisen die äußeren Enden 22 des Eckelements schlitzartige Aufnahmen 23 für eine Einschubleiste 24, die beschriftbar ist, auf.

## Patentansprüche

1. Haltevorrichtungen für Petrischalen o.dgl., mit einem die Petrischalen wenigstens teilweise umgreifenden Grundkörper (3), der im Querschnitt im wesentlichen C-förmig ausgebildet ist und eine oder mehrere übereinander gestapelte Petrischalen über einen Winkel (α) von mehr als 180° ihres Umfanges umgreift, **dadurch gekennzeichnet**, daß der Grundkörper (3) auf der zur Petrischale(n) aufnehmenden Seite abgewandten Seite eine Verbindungseinrichtung (4) zur Ankopplung einer oder mehrerer weiterer Haltevorrichtungen aufweist.

2. Haltevorrichtung nach Anspruch 1, dadurch gekennzeichnet, daß der Winkel (α) im Bereich von 182° bis 220° liegt, insbesondere 190° ist.

3. Haltevorrichtung nach einem der vorhergehenden Ansprüche, dadurch gekennzeichnet, daß sie eine Tragevorrichtung aufweist oder eine Tragevorrichtung an der Haltevorrichtung befestigbar ist.

4. Haltevorrichtung nach einem der vorhergehenden Ansprüche, dadurch gekennzeichnet, daß die Verbindungseinrichtung (4) eine Zapfen-Loch-Verbindung (5, 6) ist.

5. Haltevorrichtung nach einem der Ansprüche 1 bis 3, dadurch gekennzeichnet, daß die Verbindungseinrichtung (4) eine Nut-Feder-Verbindung ist.

6. Haltevorrichtung nach einem der vorhergehenden Ansprüche, dadurch gekennzeichnet, daß die beiden Verbindungselemente der Verbindungseinrichtung (4) in einem Winkel (β) von 0°, 60°, 90°, 108° usw., zueinander stehen.

7. Haltevorrichtung nach einem der vorhergehenden Ansprüche, dadurch gekennzeichnet, daß 21 übereinander gestapelte Petrischalen einsetzbar sind.

8. Haltevorrichtung nach einem der vorhergehenden Ansprüche, dadurch gekennzeichnet, daß sie zusammen mit wenigstens einer weiteren Haltevorrichtung eine Aufnahme (8) für ein Gestell o.dgl. bildet.

9. Haltevorrichtung nach Anspruch 8, dadurch gekennzeichnet, daß die Aufnahme (8) von einer zylinderförmigen Ausnehmung (7) gebildet wird.

10. Haltevorrichtung nach Anspruch 8 oder 9, dadurch gekennzeichnet, daß das Gestell einen vertikalen Stab und ggf. einen Drehteller aufweist.

11. Haltevorrichtung nach einem der vorhergehenden Ansprüche, dadurch gekennzeichnet, daß sie in Achsrichtung stapelbar sind.

12. Haltevorrichtung nach einem der vorhergehenden Ansprüche, dadurch gekennzeichnet, daß sie mit einem Boden (10) und/oder einem Deckel versehen sind.

13. Haltevorrichtung nach einem der vorhergehenden Ansprüche, dadurch gekennzeichnet, daß sie aus Kunststoff oder Metall hergestellt sind.

14. Haltevorrichtung nach einem der vorhergehenden Ansprüche, dadurch gekennzeichnet, daß das Material temperaturbeständig, insbesondere bis 150° C beständig ist.

15. Haltevorrichtung nach einem der vorhergehenden Ansprüche, dadurch gekennzeichnet, daß das Material einfärbbar ist oder bestimmte Farben oder Kodierungen aufweist.

16. Haltevorrichtung nach einem der vorhergehenden Ansprüche, dadurch gekennzeichnet, daß sie beschriftbar ist.

17. Haltevorrichtung nach einem der vorhergehenden Ansprüche, dadurch gekennzeichnet, daß sie ein Kennzeichnungsfeld aufweist.

18. Halteeinheit, bestehend aus zwei oder mehreren Haltevorrichtungen nach einem der vorhergehenden Ansprüche.

## Claims

1. A holding device for petri dishes, having a basic support for gripping at least part of the circumference of the petri dishes, said support having an essentially C-shaped cross section and adapted to grip one or more petri dishes, stacked one above the other, over an angle (α) of more than 180° of their circumference,
**characterised in that**
said basic support, on the side opposite to the side that receives the petri dish or petri dishes, has a connecting device for coupling together one or more holding devices.

2. Holding device in accordance to claim 1, characterised in that the angle (α) is in the range from 182° to 200°, and especially is 190°.

3. Holding device in accordance to one of the preceding claims, characterised in that it comprises a carrying device or in that a carrying device is connectable to the holding device.

4. Holding device in accordance to one of the preceding claims, characterised in that the connecting device is a peg and hole connection.

5. Holding device in accordance to one of claims 1 to 3, characterised in that the connecting device (4) is a tongue and groove connection.

6. Holding device in accordance to one of the preceding claims, characterised in that both connecting elements of the connecting device (4) have an angle (β) of 0°, 60°, 90°, 108°, etc. with respect to each other.

7. Holding device in accordance to one of the preceding claims, characterised in that 21 petri dishes stacked one above the other are insertable.

8. Holding device in accordance to one of the preceding claims, characterised in that, together with at least one further holding device, it provides a rack or the like.

9. Holding device in accordance to claim 8, characterised in that the receiving means (8) is provided by a cylindrical recess (7).

10. Holding device in accordance to claims 8 or 9, characterised in that the rack comprises a vertical rod and, as the case may be, a rotating plate.

11. Holding device in accordance to one of the preceding claims, characterised in that it can be stacked in axial direction.

12. Holding device in accordance to one of the preceding claims, characterised in that it is provided with a bottom (10) and/or a cover.

13. Holding device in accordance to one of the preceding claims, characterised in that it is made of plastic material or metal.

14. Holding device in accordance to one of the preceding claims, characterised in that the material is temperature resistant, especiallly up to 150°C.

15. Holding device in accordance to one of the preceding claims, characterised in that the material is colourable or comprises certain colours or codes.

16. Holding device in accordance to one of the preceding claims, characterised in that it can be marked.

17. Holding device in accordance to one of the preceding claims, characterised in that it is provided with a marking space.

18. Holding unit, comprising two or more holding devices in accordance to one of the preceding claims.

## Revendications

1. Dispositif support pour boîtes de Pétri ou analogues, comportant un corps de base (3) qui enserre au moins partiellement les boîtes de Pétri, qui présente, en section transversale, essentiellement la forme d'un C et qui enserre une ou plusieurs boîtes de Pétri empilées les unes sur les autres sur un angle (α) de plus de 180 ° de leur périphérie, caractérisé en ce que le corps de base (3) comporte, sur son côté opposé au côté recevant la (ou les) boîte(s) de Pétri, un dispositif de liaison (4) destiné au couplage avec un ou plusieurs autres dispositifs supports.

2. Dispositif support selon la revendication 1, caractérisé en ce que l'angle (α) est compris dans une gamme allant de 182 ° à 220 °, en particulier 190 °.

3. Dispositif support selon l'une quelconque des revendications précédentes, caractérisé en ce qu'il comporte un dispositif de transport ou qu'un dispositif de transport peut être fixé sur le dispositif support.

4. Dispositif support selon l'une quelconque des revendications 1 à 3, caractérisé en ce que le dispositif de liaison (4) comporte une liaison à tenon et mortaise (5, 6).

5. Dispositif support selon l'une quelconque des revendications précédentes, caractérisé en ce que le dispositif de liaison (4) est une liaison à encoche et ressort.

6. Dispositif support selon l'une quelconque des revendications précédentes, caractérisé en ce que les deux éléments de liaison du dispositif de liaison (4) sont disposés, l'un par rapport à l'autre, selon un angle de 0 °, 60 °, 90 °, 108° etc.

7. Dispositif support selon l'une quelconque des revendications précédentes, caractérisé en ce que l'on peut y placer 21 boîtes de Pétri empilées les unes sur les autres.

8. Dispositif support selon l'une quelconque des revendications précédentes, caractérisé en ce qu'il forme, en association avec au moins un autre dispositif support, un dispositif de réception (8) pour un bâti ou analogue.

9. Dispositif support selon la revendication 8, caractérisé en ce que le dispositif de réception (8) est constitué par un évidement cylindrique (7).

10. Dispositif support selon la revendication 8 ou 9, caractérisé en ce que le bâti est un poteau vertical et, le cas échéant, un plateau tournant.

11. Dispositif support selon l'une quelconque des revendications précédentes, caractérisé en ce qu'il peut être empilé dans la direction de son axe.

12. Dispositif support selon l'une quelconque des revendications précédentes, caractérisé en ce qu'il est muni d'un fond (10) et/ou d'un couvercle.

13. Dispositif support selon l'une quelconque des revendications précédentes, caractérisé en ce qu'il est fabriqué en matière synthétique ou en métal.

14. Dispositif support selon l'une quelconque des revendications précédentes, caractérisé en ce que la matière est résistante à la température, en particulier jusqu'à 150 °C.

15. Dispositif support selon l'une quelconque des revendications précédentes, caractérisé en ce que la matière peut être teinte ou présente des couleurs ou des codages déterminés.

16. Dispositif support selon l'une quelconque des revendications précédentes, caractérisé en ce qu'il peut recevoir des inscriptions.

17. Dispositif support selon l'une quelconque des revendications précédentes, caractérisé en ce qu'il comporte une plage pour un signe distinctif.

18. Unité support, constituée de deux ou plusieurs dispositifs supports selon l'une quelconque des revendications précédentes.
